## Europäisches Patentamt
## European Patent Office
## Office européen des brevets

(11) Veröffentlichungsnummer : **0 071 951 B1**

(12) **EUROPÄISCHE PATENTSCHRIFT**

(45) Veröffentlichungstag der Patentschrift :
30.12.86

(51) Int. Cl.⁴ : **A 61 M 1/30**

(21) Anmeldenummer : 82106985.3

(22) Anmeldetag : 03.08.82

(54) Vorrichtung zum Reinigen von Blut.

(30) Priorität : 05.08.81 DE 3131075

(43) Veröffentlichungstag der Anmeldung :
16.02.83 Patentblatt 83/07

(45) Bekanntmachung des Hinweises auf die Patenterteilung : 30.12.86 Patentblatt 86/52

(84) Benannte Vertragsstaaten :
BE FR GB IT NL

(56) Entgegenhaltungen :
DE-A- 2 441 210
DE-A- 2 513 490
DE-A- 2 636 290
DE-B- 2 522 180
US-A- 3 228 397
BRITISH MEDICAL JOURNAL, vol. 281, 25. Oktober 1980, London J. CUNNINGHAM et al., "New system for single-needle dialysis" Seiten 1109-1110

(73) Patentinhaber : Fresenius AG
Gluckensteinweg 5
D-6380 Bad Homburg (DE)

(72) Erfinder : Mathieu, Bernd, Dr.
Unten am Galgenberg 19
D-6683 Spiesen (DE)
Erfinder : Polaschegg, Hans-Dietrich, Dr.
Grünwiesenweg 9
D-6370 Oberursel (DE)

(74) Vertreter : Luderschmidt, Wolfgang, Dr. Dipl-Chem.
Görtz, Dr. Fuchs, Dr. Luderschmidt Patentanwälte
Sonnenberger Strasse 100 Postfach 26 26
D-6200 Wiesbaden (DE)

## Beschreibung

Die Erfindung betrifft eine Vorrichtung zum Reinigen von Blut von Stoffwechselprodukten, gemäß dem Oberbegriff des Anspruchs 1.

Im Gegensatz zur üblichen Hämodialyse, bei der ein Patient jeweils mit einer Nadel für den Hin- und Rücklauf des Blutes punktiert wird, kommt man bei der Single-Needle-Dialyse mit nur einer Nadel aus. Diese Methode hat den Vorteil, daß sich die Zahl der Punktionen auf die Hälfte reduziert, so daß einerseits die Gefäße geschont und andererseits die bei der Punktion auftretenden Schmerzen vermindert werden. Insbesondere bei Akut-Dialysen genügt ein einziger Gefäßzugang, wodurch die Behandlung im Notfall erheblich erleichtert wird.

Nachteilig bei diesem Single-Needle-Verfahren sind die diskontinuierliche Steuerung der Blutzuführung und der -abfuhr sowie die teilweise Vermischung von gereinigtem und ungereinigtem Blut im Bereich der Nadel, mit der der Patient an die Dialysevorrichtung im Single-Needle-Verfahren angeschlossen wird.

Im Gegensatz zum kontinuierlichen Dialyseverfahren mit der Zwei-Nadel-Methode, bei der der im Dialysesystem herrschende Druck im wesentlichen genau eingestellt werden kann, tritt bei dem Single-Needle-Verfahren ein stetigen Druckaufbau und -abfall auf, der durch den wechselweisen Betrieb der Blutzufuhr und -abfuhr bedingt ist. Insofern ist es bei dem Single-Needle-Verfahren schwierig, die gewünschten Förderraten bei einem bestimmten mittleren Druck einzuhalten. Es wurden daher Versuche unternommen, diese Nachteile beim Single-Needle-Verfahren auszuschalten.

Aus der DE-OS-24 17 900 ist eine Vorrichtung zum Dialysieren von Blut bekannt, bei der unmittelbar hinter einer y-förmigen Nadel, deren Enden mit der Arterien- und Venenleitung verbunden sind, eine Pumpe, eine Dialysemembran und ein Blasenabscheider in der Venenleitung angeordnet sind. Dieser Blasenabscheider ist über eine weitere Leitung mit einem Druckmonitor verbunden, der eine unmittelbar am y-förmigen Ende der Nadel befindliche Doppel-Klemmeinrichtung zum wahlweisen Abklemmen der Arterienleitung bzw. der Venenleitung steuert. In dieser Anordnung erfolgt eine druckabhängige Steuerung in einem Blasenabscheider mit starren Wänden dadurch, daß das in den Blasenabscheider geförderte Blut den darüberliegenden Luftraum elastisch zusammendrückt und somit den Druckmonitor auslöst, der wiederum die Doppel-Klemmeinrichtung steuert.

Eine derartige Anordnung ist insofern nachteilig, als einerseits die für die Drucksteuerung erforderlichen Grenzwertmanometer teuer und störanfällig sind, andererseits die an der Dialysemembran auftretenden Druckschwankungen den Flüssigkeitsdurchgang durch die Membran erheblich stören und damit eine bilanzierte Flüssigkeitsentnahme durch den Dialysator erschwert bzw. unmöglich gemacht wird. Hinzu kommen bei einer derartigen druckgesteuerten Anordnung die Gefahr des Zurücksaugens von evtl. kontaminierter Flüssigkeit und des Ansaugens von Luft durch Undichtigkeiten im Leitungssystem.

Aus der DE-OS-26 36 290 ist eine Vorrichtung zur Steuerung und Überwachung des Blutflusses bei der Blutdialyse bekannt, bei der anstelle des vorstehenden Blasenabscheiders eine Meßkammer vorgesehen ist, die an ihren ebenfalls starren Seitenwänden Sensoren aufweist, durch die das in der Meßkammer befindliche Blutvolumen zwischen einem oberen und unteren Grenzwert gesteuert werden kann. An ihrem oberen Ende ist diese Meßkammer mit einer den Druckausgleich herbeiführenden Einrichtung, beispielsweise einem Sterilfilter oder einem Beutel oder Ballon derart verbunden, daß die gesamte Meßkammer im wesentlichen druckentlastet ist. Infolge dieser Druckentlastung muß diese bekannte Anordnung zwei Pumpen aufweisen, wobei die eine für die Blutzufuhr und die andere für die Blutabfuhr dient und vor bzw. hinter der Dialysemembran angeordnet sind. Ein Einsatz von erheblich billigeren Klemmen, durch die eine Pumpe eingespart werden kann, ist demnach nicht möglich, so daß die Herstellungskosten einer derartigen Dialysevorrichtung erheblich größer sind. Diese Kosten werden noch dadurch gesteigert, daß an die Dialysemembran selbst auf ihrer Dialysatseite ein Unterdruck durch eine spezielle Vorrichtung erzeugt werden muß, um dem Patienten die gewünschte Flüssigkeitsmenge zu entziehen.

Zur Behebung dieser Schwierigkeiten wurde im British Medical Journal, vol. 281 (1980), S. 1109, ein Single-Needle-Dialysesystem vorgeschlagen, bei dem in der zur Dialysemembran führenden Arterienleitung eine Blutzuführungspumpe, ein nachgiebiger Speicher und eine Blutabführungspumpe vorgesehen sind. In der Venenleitung ist lediglich eine Klemme zum Öffnen und Schließen dieser Leitung vorgesehen. Beim Betrieb dieses Systems wird der Speicher zunächst durch die eine Pumpe vollgepumpt, ohne daß sich in diesem ein Druck aufbaut. Nach dem Abschalten dieser Pumpe wird die andere Pumpe eingeschaltet und zugleich die Klemme in der Venenleitung geöffnet. Das im Speicher befindliche Blut wird an der Dialysemembran vorbei durch die Venenleitung in den Patienten zurückgepumpt, wobei der in dieser Anordnung herrschende Druck allenfalls um weniger als 10 mm Hg schwankt. Da während des Füllvorganges des Speichers kein Druck erzeugt wird, wird zwangsläufig wiederum eine zweite Pumpe benötigt, mit der das Blut durch das Dialysesystem befördert wird. Weiterhin muß der an der Dialysemembran einzustellende Transmembrandruck extern gesteuert werden, wodurch die Apparatekosten erheblich steigen und ein Betrieb der gesamten Vorrichtung schwierig wird.

Aus der DE-A-2 522 180 ist eine übliche Hämodialysevorrichtung mit zwei Anschlüssen bekannt, bei der der Ultrafiltrationsdruck mit Hilfe eines als doppelwandiges Rohr ausgebildeten Druckhalteventils gesteuert wird. Dieses Druckhalteventil dient somit nicht als Zwischenspeicher, sondern vielmehr als Einrichtung zur Aufrechterhaltung der Druckkonstanz.

Der Erfindung liegt deshalb die Aufgabe zugrunde, eine Vorrichtung der eingangs erwähnten Art zu schaffen, bei der sowohl eine Druck- als auch Volumensteuerung möglich ist, mit nur einer Pumpe gearbeitet werden kann und — sofern mit Drucksteuerung gearbeitet wird — ein im wesentlichen konstanter Druck an der Dialysemembran beim Blutdurchgang anliegt.

Die Lösung dieser Aufgabe erfolgt durch die kennzeichnenden Merkmale des Anspruchs 1.

Erfindungsgemäß wird ein Speicher eingesetzt, dessen Aussenwände elastisch erweiterbar sind, so daß sich das Gesamtvolumen des Speichers um ein Vielfaches vergrößern kann. Infolge seiner elastischen Erweiterung baut sich im Speicher ein auf die eingespeiste Blutmenge wirkender Druck auf, der vom Volumen des eingespeisten Blutes abhängig ist.

Die Druckcharakteristik hängt natürlich von der Länge und dem Querschnitt des Druckspeichers sowie insbesondere von der Wandstärke des Speichers ab. Üblicherweise kann ein Speicher bei den gewünschten Abmessungen das Zwanzigfache und mehr seines Ausgangsvolumens aufnehmen, ohne daß es zu einem abrupten steilen Druckanstieg käme, der zu einem Zerreißen des Speichers führen würde. Die Druckcharakteristik des Schlauchs selbst sieht so aus, daß der Druck nach dem drucklosen Auffüllen des Schlauchs bei weiterer Blutzufuhr steil bei wenig weiterem zugeführten Blutvolumen ansteigt und anschließend auf einen Wert einschwenkt, der beispielsweise bis zum Zwanzigfachen der zugeführten Blutmenge nur noch gering ansteigt oder sogar im wesentlichen konstant bleibt. Eine über diese Volumenvergrößerung hinausführende Blutzufuhr führt jedoch zu einem steilen Anstieg des Druckes bei wenig zusätzlich zugeführtem Blutvolumen. Ein derart unter Druck stehender Speicher kann einerseits leicht überwacht werden, da die oberen und unteren Druckwerte infolge der vorstehend genannten Charakteristik schon bei einer geringfügigen Volumenänderung im Grenzbereich über- oder unterschritten werden, so daß hierdurch eine Drucksteuerung erheblich erleichtert wird. Hierdurch entfällt die bei anderen Single-Needle-Vorrichtungen übliche Mittelwertsteuerung des Druckes. Weiterhin wird im Bereich des Druckplateaus eine im wesentlichen gleichen Druckbereich befindliche Blutmenge abgegeben, so daß der an der Dialysemembran anliegende Druck im wesentlichen gleich ist und daher keine Unregelmäßigkeiten bei der Dialyse zu befürchten sind.

Überdies entfällt eine der beiden Pumpen in diesem Single-Needle-System, da es sich bei dem Speicher um einen unter Druck stehenden, sich selbst entladenden Speicher handelt, der lediglich zum Füllen eine Pumpe benötigt, während das Entladen des Speichers durch eine Klemme gesteuert werden kann.

Da sich das Speichervolumen beim Einspeisungsvorgang erheblich vergrößert, kann natürlich auch eine Volumensteuerung des Speichers stattfinden, wozu beispielsweise optische oder mechanische Steuereinrichtungen herangezogen werden können. Eine derartige Steuereinrichtung kann wahlweise zur Drucksteuerung oder anstatt dessen eingesetzt werden.

Bei dem erfindungsgemäßen Speicher handelt es sich um einen Billigartikel, der vorteilhafter Weise aus handelsüblichen Latexschläuchen gefertigt wird und somit keine produktionstechnischen Schwierigkeiten aufwirft. Er ist leicht zu sterilisieren und kann in Kombination mit dem üblichen Dialyseschlauchsystem gefertigt und vertrieben werden.

Weitere Einzelheiten, Vorteile und Ausführungsformen sind in der nachfolgenden Beschreibung unter Bezugnahme auf die Zeichnung erläutert.

Es zeigen

Figur 1 schematisch eine Single-Needle-Dialyseanordnung mit dem erfindungsgemäßen Speicher

Figur 2 einen Längsschnitt durch eine erste Ausführungsform des erfindungsgemäßen Speichers

Figur 3 einen Längsschnitt durch eine zweite Ausführungsform des erfindungsgemäßen Speichers

Figur 4 eine Druck/Volumen-Charakteristik der in Fig. 3 gezeigten Ausführungsform beim Befüllen

Figur 5 ein weiteres Schema einer Vorrichtung zur Dialyse unter Einsatz des erfindungsgemäßen Speichers

Figur 6 den Endabschnitt einer dritten Ausführungsform des erfindungsgemäßen Speichers im Längsschnitt

Figur 7 den Endabschnitt einer vierten Ausführungsform des erfindungsgemäßen Speichers im Längsschnitt und

Figur 8 den Endabschnitt einer fünften Ausführungsform des erfindungsgemäßen Speichers teilweise im Längsschnitt.

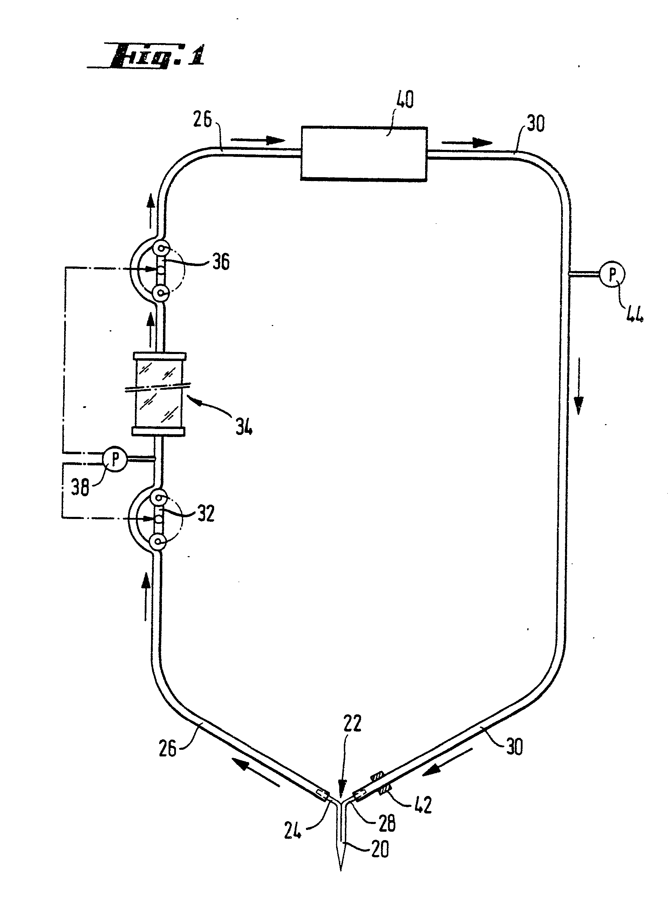

Gemäß Fig. 1 wird einem Patienten über eine Hohlkanüle 20 Blut entnommen, die in ein Blutgefäß eingeschoben wird. Diese Hohlkanüle 20 weist an ihrem Ende einen y-förmigen Anschluß 22 auf, bei dem der eine Anschluß 24 mit der Arterienleitung 26 verbunden ist, während der andere Ast 28 mit der Venenleitung 30 verbunden ist. Die Hohlkanüle kann als Biflownadel ausgebildet sein, d. h. sie besteht aus zwei ineinander koaxial angeordneten Kanülen, bei denen die äußere Kanüle um wenige Millimeter zurückgesetzt ist, um einen Vermischungseffekt zu vermeiden. Andrerseits kann jedoch auch der geringe Vermischungseffekt bei der vorzugsweise eingesetzten y-förmigen Nadel 20 hingenommen werden.

In der Arterienleitung 26 wird das Blut von der y-förmigen Hohlnadel 20 mit Hilfe einer Pumpe 32 angesogen, die vorteilhafter Weise als Schlauchpumpe ausgebildet ist. An diese Pumpe 32 schließt sich in der Arterienleitung 26 der Speicher 34 an, in dem das durch die Pumpe 32 angeförderte Blut zwischengespeichert werden kann. Hinter dem Speicher 34 befindet sich in dieser ersten Ausführungsform eine zweite Pumpe 36, die dann ihre Tätigkeit aufnimmt, wenn die Pumpe 32 abgeschaltet wird.

In der Arterienleitung 26 ist weiterhin zwischen der ersten Pumpe 32 und der zweiten Pumpe 36 ein Sensor 38 vorgesehen, dessen Signal zum wechselweisen Betrieb der ersten und zweiten Pumpe 32 bzw. 36 herangezogen wird und der vorzugsweise als Drucksensor ausgebildet ist.

Hinter der zweiten Pumpe 36 ist die Arterienleitung mit der Dialysemembran 40 verbunden, in der das zu reinigende Blut von den Stoffwechselprodukten und überschüssiger Flüssigkeit befreit wird.

Das gereinigte Blut wird über die Venenleitung 30 zum einen Ast 28 der Hohlkanüle 20 zurückgeführt, wobei diese Venenleitung 30 in der Nähe des Astes 28 durch eine Klemme 42 geschlossen werden kann, die ein Ansaugen von Blut aus der Venenleitung 30 beim Betrieb der Pumpe 32 verhindert.

Weiterhin kann in der Venenleitung 30 ein Drucksensor 44 angeordnet sein, der den Druck anzeigt, der sich in dem Leitungsabschnitt zwischen der Pumpe 36 und der Klemme 42 aufbaut.

Die in Fig. 1 gezeigte erste Ausführungsform wird folgendermaßen betrieben :

Das Blut wird über die Hohlkanüle 20 mittels der Pumpe 32 angesaugt, wobei die Klemme 42 und die Pumpe 36, die als Klemme wirkt, geschlossen bzw. nicht in Betrieb sind. Hierdurch wird zwangsläufig der schlauchförmig ausgebildete Speicher 34 zunächst drucklos aufgefüllt. Anschliessend erweitern sich unter sprunghaftem Druckanstieg seine Außenwände ähnlich dem Zustand, in dem ein Ballon aufgeblasen wird. Es muß dabei nur eine geringe Menge Blut zugeführt werden, um diesen sprunghaften Druckanstieg in ein nur noch geringfügig ansteigendes Druckplateau umzuwandeln, d. h. während der Speicher 34 weiter mit Blut angefüllt wird, bleibt der Druck im Speicher praktisch gleich, ohne zunächst wieder sprunghaft anzusteigen. Dieser Anstieg erfolgt dann, wenn ein Grenzzustand erreicht wird, bei dem sich die elastischen Eigenschaften der Speicherwand verändern.

Damit der Speicher 34 nicht zerplatzt, ist — wie vorstehend festgestellt — zwischen der ersten Pumpe 32 und der zweiten Pumpe 36 ein Sensor 38 vorgesehen, der in der vorliegenden Ausführungsform als Drucksensor ausgebildet ist und bei einem bestimmten erreichten Druckwert die Pumpe 32 ausschaltet und zugleich die Pumpe 36 anschaltet und die Klemme 42 öffnet. Hierdurch wird das in dem Speicher 34 befindliche Blut vollständig abgepumpt und über die Dialysemembran 40 in die Venenleitung 30 und von dort über die Hohlkanüle 20 in den Patienten zurückgefördert. Wenn in der Venenleitung 30 ein unterer Druck unterschritten wird, was beispielsweise durch den Drucksensor 44 oder den Sensor 38 angezeigt werden kann, werden die Pumpe 36 abgeschaltet und die Klemme 42 geschlossen und die Pumpe 32 wieder angeschaltet, so daß der Zyklus von neuem beginnt.

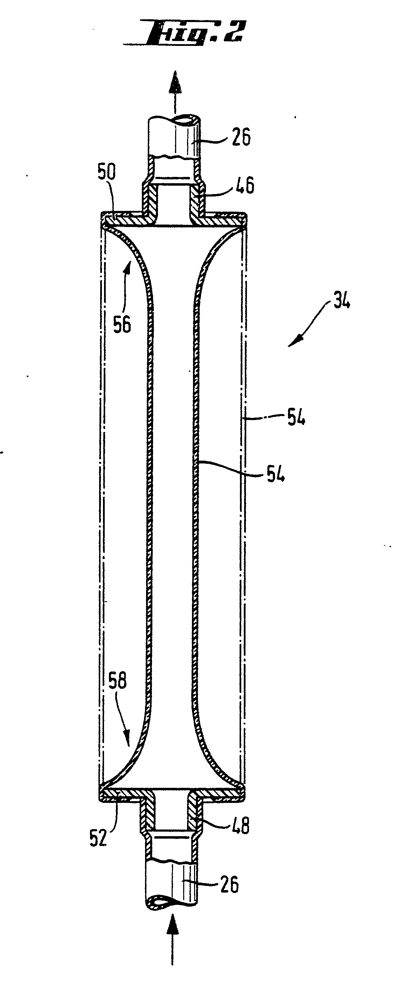

In Fig. 2 ist die erste Ausführungsform des erfindungsgemäßen Speichers 34 im Längsschnitt gezeigt. Dieser Speicher 34 weist an seinen beiden Enden Anschlüsse 46 und 48 auf, auf die die üblicherweise aus flexiblem Kunststoff bestehende Arterienleitung 26 aufgeschoben und befestigt wird. Diese Anschlüsse 46 und 48 können in einer nicht gezeigten Halterung der Dialysevorrichtung angebracht und dort befestigt werden. Die Anschlüsse 46 und 48 befinden sich jeweils auf einer Platte 50 und 52, die vorteilhafterweise eine kreisförmige Form aufweist. Zwischen diesen Platten 50 und 52 befindet sich der eigentliche Speicherkörper, der vorteilhafterweise als elastischer Schlauch 54 ausgebildet ist. In der in Fig. 2 gezeigten Ausführungsform steht der Schlauch 54 lediglich in seinen Endbereichen 56 und 58 unter Zugspannung, da er auseinandergezogen werden muß, um über die Platten 50 und 52 überstülpt werden zu können. An diesen Platten 50 und 52 ist er beispielsweise durch Kleben oder mechanisches Klemmen so befestigt, daß er gegen jedes Durchschlagen von Blut gesichert ist. Entsprechend dieser Ausführungsform ist also die Querschnittsfläche des Schlauchs 54 geringer als die Fläche der Platten 50 und 52. Andererseits muß dies jedoch nicht zwangsläufig der Fall sein. Diese Flächen können ebenso gleich sein oder es kann sogar die Querschnittsfläche des Schlauchs 54 größer sein als die Oberfläche der Platte 50 oder 52.

Infolge seiner hochelastischen Eigenschaften kollabiert der Schlauch 54 im nicht aufgespannten Zustand und kann somit durch Anlegen von geringem Unterdruck völlig evakuiert werden. Insofern ist in dieser ersten Ausführungsform des Speichers 34 das Befestigen der Anschlüsse 46 und 48 an einer Dialysevorrichtung notwendig, da der Schlauch 54 nicht ausreichend eigenstabil ist.

Als Material für diesen Schlauch 54 werden hochelastische Materialien eingesetzt, beispielsweise organische Polymere und deren Gemische, wie Polyurethane, Neoprene, Kautschuke, Silikonkautschuke, Latex, Gummi, regenerierter Gummi u. dgl. Vorzugsweise wird Latex eingesetzt, das ggf. übliche Zusatz- und die Elastizität verbessernde Mittel aufweist. Ein derartiger Schlauch kann wenigstens um das Fünffache seines Lumens, insbesondere um das Zehnfache ausgedehnt werden, so daß er also mindestens in der bevorzugten Ausführungsform das Zehnfache seines Innenvolumens zusätzlich aufnehmen kann.

Die Wandstärke eines derartigen Schlauches liegt in einem Bereich von etwa 0,05-0,5, vorzugsweise 0,1-0,4 mm. Sie wird in Abhängigkeit von der gewünschten Druckcharakteristik und der Volumenerweiterung des einzusetzenden Speichers 34 gewählt. Je dicker die Wandstärke des Schlauchs 54 ist, desto höher ist der Anfangsdruck, der aufzuwenden ist, um zu dem über einen weiten Volumenbereich führenden Druckplateau zu kommen. Vorzugsweise wird die Wandstärke so gewählt, daß das Druckplateau oberhalb des in der Vene herrschenden Drucks, vorzugsweise über 20 mm Hg liegt. Dieser Druck muß also in der Venenleitung vorliegen, um überhaupt Blut in die Vene überführen zu können.

Das Schlauchlumen und die Länge des Schlauches richten sich nach dem Einsatzzweck sowie der Auslegung der Dialysevorrichtung. Üblicherweise wird der Durchmesser eines Schlauchs in einem Bereich von 5-15, vorzugsweise etwa 8 mm liegen, während die Länge des Schlauchs 10-20, vorzugsweise ca. 15 cm betragen kann. Es sind jedoch aber auch kürzere oder längere Abmessungen denkbar, sofern dies der Einsatzzweck erfordert. Wenn nur geringe Blutvolumina befördert werden, wird man einen entsprechend geringer dimensionierten Speicher mit einem entsprechend kleineren Schlauch einsetzen als bei größeren Blutumsätzen.

Ein im wesentlichen ebenes Druckplateau nach dem abrupten Anstieg erhält man vorzugsweise dadurch, daß die Wandstärke des Schlauchs über seine Gesamtlänge im wesentlichen gleich ist. Diese Ausführungsform ist in der Fig. 2 gezeigt.

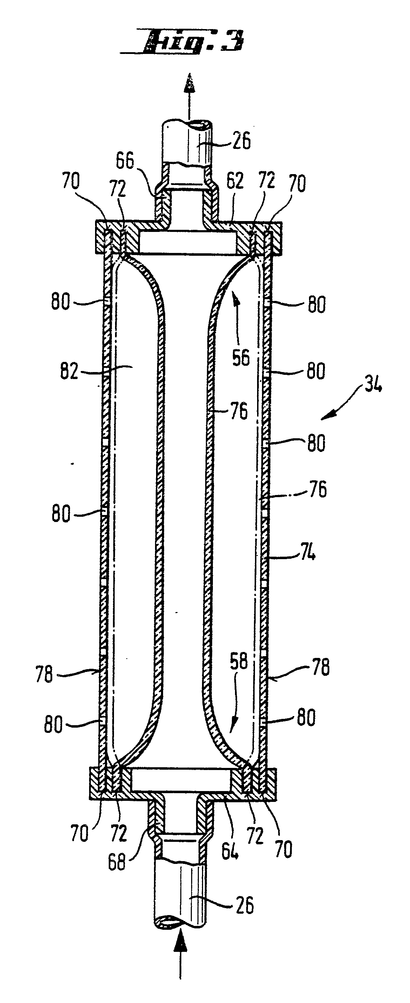

In Fig. 3 ist eine weitere Ausführungsform gezeigt, bei der die Wandstärke des Schlauchs 76 als Speicherkörper über seine Gesamtlänge im wesentlichen stetig abnimmt. Ein derartiger Schlauch kann dadurch erzeugt werden, daß man einen Stab in eine flüssige Latexmasse eintaucht und diesen Stab anschließend aus der flüssigen Latexmasse herauszieht. Durch den flüssigen Zustand dieser Latexmasse fließt ein Teil der an dem Schlauch hängenden Masse von oben nach unten, so daß die Wandstärke des dadurch hergestellten Schlauchs vom einen Schlauchende zum anderen Schlauchende stetig zunimmt. Hierdurch steigt das Druckplateau nach dem abrupten Anstieg ebenfalls stetig an.

In der in Fig. 3 gezeigten Ausführungsform des erfindungsgemäßen Speichers 34 ist wiederum ein Schlauch 76 vorgesehen, der zwischen zwei Platten 62 und 64 fest eingespannt ist. Diese Platten 62 und 64 weisen wiederum zwei Anschlüsse 66 und 68 für den Arterienschlauch 26 auf. Jeweils auf der Unterseite der Platten sind zwei ringförmige Nuten 70 und 72 vorgesehen. In die Nut 70 wird ein starres Gehäuse 74 eingepaßt, dessen Querschnitt erheblich über dem Querschnitt des Schlauchs 76 liegt, der in die zweite Nut 72 fest eingepaßt wird. Das Einpassen der Schläuche kann — wie vorstehend erläutert — durch Einkleben oder durch mechanisches Befestigen erfolgen.

Der Schlauch 76, der als Speicherkörper dient, wird wie der Speicherkörper 54 der ersten Ausführungsform des Speichers 34 durch Ausdehnung in die Innennut 72 gebracht. Dabei entsteht zwischen dem Schlauch 76 und dem starren Gehäuse 74 ein Luftraum 82, der vorzugsweise durch wenigstens eine die Wand 78 des Gehäuses 74 durchsetzende Öffnung 80, insbesondere eine Vielzahl davon belüftbar ist, so daß kein Gegendruck bei der Ausweitung des Schlauchs in diesem Raum 82 auftritt.

Sofern nur eine Öffnung 80 vorgesehen ist und eine Schlauch 76 mit unterschiedlicher Wandstärke eingesetzt wird, ist diese Öffnung 80 im Gehäuse 74 im Bereich der dickeren Wandstärke des Schlauchs 76 vorgesehen, da in diesem Bereich die Aufblähung des Schlauchs 76 zuletzt erfolgt.

Andrerseits kann es jedoch vorteilhaft sein, diesen Raum 82 ohne Druckausgleich zu halten, d. h. keine Löcher im starren Gehäuse 74 vorzusehen. Hierdurch kann einerseits erreicht werden, daß der Schlauch 76 bereits im nicht gefüllten Zustand vollständig evakuiert und zusammengepreßt vorliegt, so daß bereits bei der ersten Befüllung ein Gegendruck auftritt. Durch das zusätzliche Vorsehen eines elastischen, im Raum 82 eingeschlossenen Luftpolsters wird die Elastizität des Speichers 34 erhöht, was in manchen Anwendungsfällen besonders vorteilhaft sein kann.

Zweckmäßigerweise werden die Abmessungen des Gehäuses 74 so gewählt, daß der Raum 82 etwa 50-80 ml beträgt, während der elastische Schlauch im drucklosen Zustand etwa 10-15 ml faßt.

Hinzuzufügen ist, daß als Material für den elastischen Schlauch 54 bzw. 76 alle wasserundurchlässigen Materialien in Frage kommen, die eine entsprechend große Bruchdehnung (> 200 %) aufweisen, wozu die vorstehend genannten Materialien gehören.

Wenn bei dieser Ausführungsform Blut in den Schlauch 76 hineingepumpt wird und die Pumpe 36 außer Betrieb ist, steigt der Druck anfangs nur geringfügig an, da der Schlauch sich unter geringer Dehnung füllt und dabei das Volumen nur geringfügig zunimmt. Danach bläht sich der Schlauch an einer Stelle solange auf, bis er am Gehäuse 74 anliegt. Die sich dann ausbildenden Dehnungsfronten bewegen sich in axialer Richtung bis zum Ende des Gehäuses 74, wobei die im Raum 82 befindliche Luft durch die Öffnungen 80 verdrängt wird. Beim Laufen der Dehnungsfronten in axialer Richtung bleibt der Druck auf das einzupumpende Blut im wesentlichen gleich, sofern die Wandstärke des Schlauchs 76 im wesentlichen einheitlich ist. Hierdurch kommt das vorstehend beschriebene im wesentlichen konstandte Druckniveau zustande.

Wenn der Raum 82 vollständig durch den Schlauch 76 ausgefüllt ist, so daß dieser an der Innenwand des Gehäuses 74 anliegt, steigt der Druck sehr rasch an, da sich das Gehäuse 74 nur sehr schwer verformen läßt.

Hier setzt die Betätigung der Pumpe 36 durch einen entsprechenden Sensor 38 ein, der zugleich die Pumpe 32 abschaltet. Dadurch wird das in den Schlauch 76 eingespeiste Blut wieder entnommen und in die Venenleitung 30 eingeführt. Beim Unterschreiten eines unteren Grenzwertes, der beispielsweise durch den Sensor 44 festgestellt werden kann, wird dieser Zyklus abgeschaltet und von neuem die Pumpe 32 betätigt.

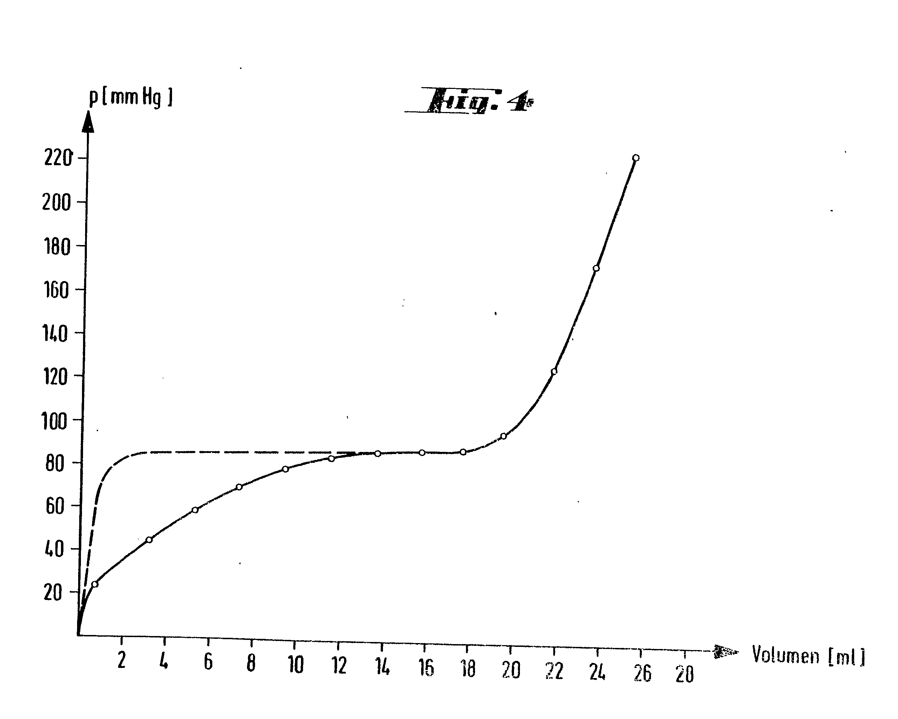

In Fig. 4 ist die Druck/Volumen-Charakteristik eines Beispiels dieser zweiten Speicherausführungsform in durchgezogener Linie gezeigt, während in gestrichelter Linie das Idealverhalten eines Schlauchs 76 mit gleichbleibender Wandstärke dargestellt ist. Das Diagramm bezieht sich natürlich auf das Blutvolumen, das bereits einem bei Normaldruck gefüllten Schlauch 76 zugeführt wird. Der gem. Fig. 4 eingesetzte Schlauch ist durch Tauchen eines Stabes in ein Latexbad und langsames Herausziehen dieses Stabes hergestellt worden, was aus der stetig ansteigenden Druckcharakteristik in Abhängigkeit vom zugeführten Blutvolumen zu ersehen ist.

Da sich im elastischen Schlauch 54 und 76 sowohl die Druck- als auch die Volumenverhältnisse verändern, können beide Parameter zur Steuerung des Speichers 34 unabhängig voneinander oder zusammen eingesetzt werden.

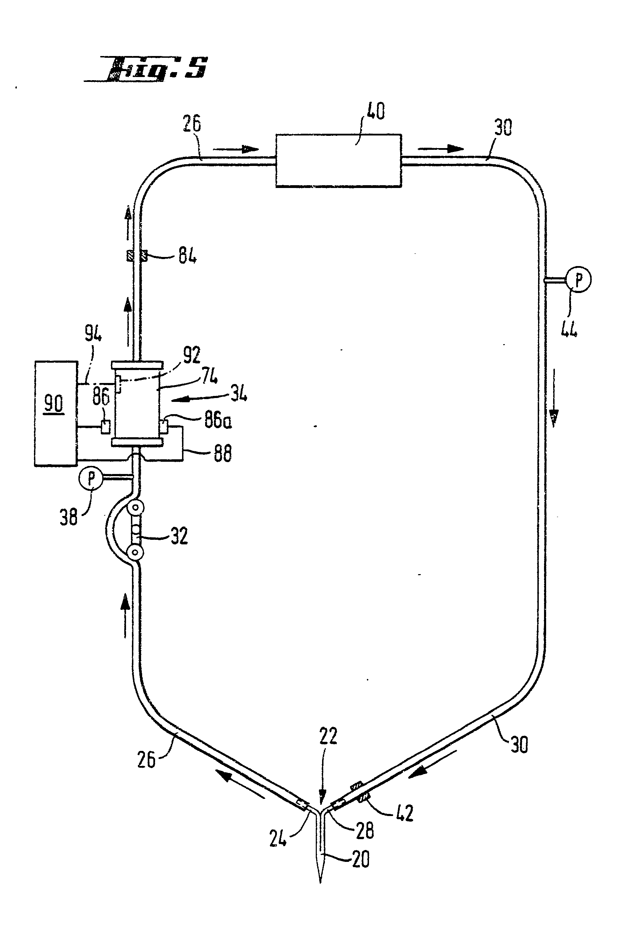

In Fig. 5 ist eine spezielle Abwandlung des in Fig. 1 gezeigten Blockschaltbildes dargestellt. Dabei ist die Pumpe 36 durch eine Klemme 84 ersetzt, die geöffnet wird, wenn einer der vorstehend genannten Parameter seinen oberen Grenzwert erreicht hat. Diese Klemme 84 kann wahlweise einen konstanten Öffnungsquerschnitt oder aber auch einen variablen Öffnungsquerschnitt aufweisen, was von der durch die Klemmenbetätigung durchzulassenden Blutmenge abhängt. Bei im wesentlichen konstantem Druckplateau wird man eine Klemme 84 mit konstantem Öffnungsquerschnitt einsetzen, um gleiche Förder- und Druckverhältnisse in der Venenleitung 30 herzustellen. Andrerseits wird man bei einem ansteigenden Druckplateau, also bei über die Öffnungszeit der Klemme 84 variierenden Druck- und Förderverhältnissen, eine Klemme 84 mit variablem Öffnungsquerschnitt einsetzen, um die durch die Venenleitung 30 geförderten Mengen im wesentlichen konstant zu halten.

Eine derartige Anordnung hat den Vorteil, daß der an der Dialysemembran anliegende Druck nicht identisch ist mit dem Pumpdruck im Speicher, so daß die Dialysemembran mit erheblich niedrigeren, beliebig gegenüber dem Speicherdruck einstellbaren Drücken betrieben werden kann.

In Fig. 5 ist weiterhin ein am Außenumfang des Speichers 34, insbesondere am Außenumfang des Gehäuses 74 angeordneter optischer Sensor 86 und 86a gezeigt, die über eine Leitung 88 mit einem Steuergerät 90 verbunden sind, das über nicht gezeigte Leitungen die Pumpe 32 bzw. die Klemme 84 und die Klemme 42 steuert. Dieses Steuergerät kann andrerseits auch mit den Sensoren 38 und 44 verbunden sein. Der optische Sensor 86 und 86a ist derart am Außenumfang des Gehäuses 74 angeordnet, daß der als Sender dienende Sensor 86 den Raum 82 durchstrahlt, und der dadurch erzeugte Strahl auf den als Detektor dienenden Sensor 86a fällt. Dieser Strahl wird durch den sich ausweitenden Schlauch 76 unterbrochen, was über das Steuergerät 90 zu einem Abschalten der Pumpe 32 bzw. der Klemme 84 und der Klemme 42 führt.

In einer weiteren Ausführungsform kann der optische Sensor 86, 86a durch einen Mikroschalter 92 ersetzt sein, der über eine Leitung 94 mit dem Steuergerät 90 verbunden ist. Dieser Schalter 92 ist ebenfalls auf der Wand des Gehäuses 74, vorzugsweise auf der Innenwand vorgesehen und wird durch die über den Mikroschalter 92 laufende Dehnungsfront des Schlauchs 76 betätigt.

Über derartige Volumen- oder Drucksteuerungen läßt sich natürlich die im Speicher 34 gespeicherte Blutmenge beliebig feststellen, sofern die eingesetzten Sensoren vorher entsprechend justiert und geeicht wurden. Weiterhin kann hierdurch eine bestimmte Blutmenge zyklisch durch die Dialysemembran 40 gepumpt werden, wobei der Speicher 34 nicht vollgepumpt werden muß.

Der Speicher 34 kann natürlich auch hinter der Dialysemembran 40, d. h. in der Venenleitung 30 angeordnet sein. Ebenso kann die Pumpe 36 bzw. die Klemme 84 unabhängig von der Anordnung des Speichers 34 ebenfalls in der Venenleitung 30 angeordnet werden, sofern dies zweckmäßig erscheint. Sofern die Pumpe 32 stetig läuft und nicht ein- und ausgeschaltet wird, ist vor ihr in der Arterienleitung 26 eine der Klemme 42 entsprechende nicht gezeigte Klemme angeordnet, die wechselweise zur Klemme 42 betätigt wird. Überdies kann die Klemme 42 entfallen, sofern die Pumpe 36 in der Venenleitung 30 vorgesehen ist, wobei die Pumpen 32 und 36 wechselweise gesteuert werden. In einer speziellen Ausführungsform können in der Arterienleitung die Pumpe 32 und in der Venenleitung die Klemme 42 gemäß dem in Fig. 1 gezeigten Blockschaltbild angeordnet sein, wobei die Pumpe 36 wegfällt, sofern der erfindungsgemäße Speicher 34 eingesetzt wird.

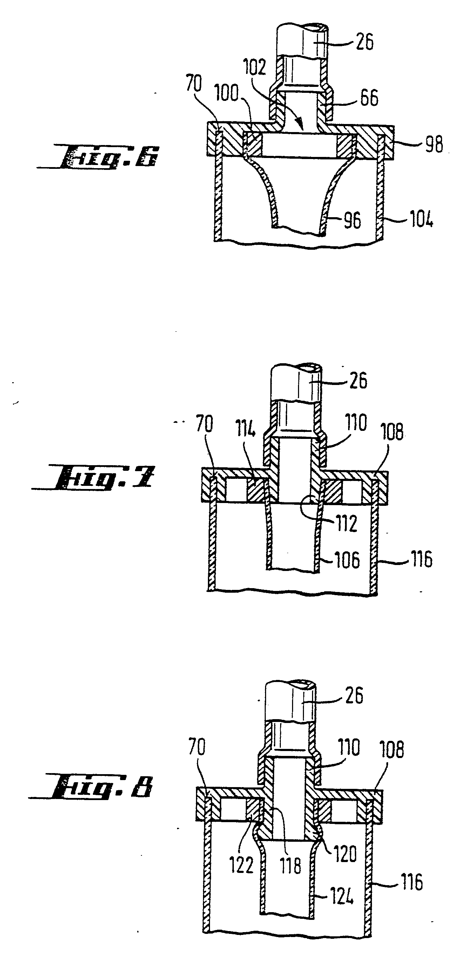

In den Fig. 6 bis 8 sind weitere Ausführungsformen des erfindungsgemäßen Speichers gezeigt, in denen speziell die Befestigung des aufblähbaren Schlauchs an den Endplatten erläutert ist.

Gemäß Fig. 6 ist der Schlauch 96 an der Endplatte 98 mit Hilfe eines Klemmrings 100 befestigt, der vorzugsweise eine konusförmige Gestalt aufweist. Vorteilhafterweise besitzt der Klemmring 100 einen Querschnitt, der größer ist als der Querschnitt des Schlauchs 96, so daß dieser unter Querdehnung über den Klemmring gezogen werden muß. Diese Anordnung wird dann in die Endplatte 98 bzw. die in der Endplatte 98 vorgesehene Ausnehmung 102 eingesetzt, wo sie infolge des hohen Reibkoeffizienten des Schlauchmaterials fest haftet. Da der Schlauch 96 sehr gut in axialer Richtung gedehnt werden kann, ist auch das starre Gehäuse 104 leicht in die Endplatte 98 einzusetzen, wobei dieses Einsetzen bereits in der Beschreibung zu der in Fig. 3 gezeigten Ausführungsform erläutert worden ist, worauf Bezug genommen wird. Im übrigen entsprechen die in Fig. 6 gezeigte Endplatte 98 sowie deren Ausgestaltung der in Fig. 3 gezeigten Platte 62 bzw. 64.

In Fig. 7 ist eine weitere Befestigungsart des Schlauchs 106 gezeigt. Dabei weist die Innenplatte 108 auf ihrer Innenseite einen mit dem Anschluß 110 fluchtenden Rohransatz 112 auf, der sich von der Platte 108 weg verjüngt. Auf diesen Rohransatz 112 ist der Schlauch 106 mit Hilfe eines Klemmrings 114 befestigt, wobei wiederum die aus dem Schlauch 106 und aus dem Klemmring 114 bestehende Anordnung auf diesen Rohransatz 112 formschlüssig aufgeschoben wird. Dabei entspricht die Befestigung des starren Genäuses 116 der bezüglich Fig. 6 beschriebenen Anordnung.

In Fig. 8 ist eine Abwandlung der in Fig. 7 erläuterten Ausführungsform dargestellt. Der Rohransatz 118 weist an seinem unteren Ende einen olivenförmigen Wulst 120 auf, dessen Außenumfang etwas größer ist als der Innenumfang des Klemmrings 122. Dieser Klemmring 122 ist jedoch so elastisch, daß er zusammen mit dem Schlauch 124 über den Wulst 120 geschoben werden kann und durch den Wulst 120 fest in Stellung gehalten wird.

Weiterhin ist es bevorzugt, daß der Innenschlauch axial vorgespannt ist, wenn der Speicher 34 in Verbindung mit dem starren Gehäuse 74, 104, 116 bzw. 124 vorliegt. Vorteilhafterweise wird der Schlauch um mindestens etwa 10 % seiner Gesamtlänge in Axialrichtung gedehnt, um ein Zusammenfallen zu verhindern. In Verbindung mit der vorstehend beschriebenen Querdehnung wird dadurch ein voll aufgespannter Schlauch erzeugt, wodurch sowohl eine bessere Aufblähbarkeit des Schlauchs im Betrieb erreicht, als auch ein Zusammenkleben des Schlauchs durch fortschreitende Vulkanisierung im Lagerzustand verhindert wird. Hinzuzufügen ist, daß das im wesentlichen starre Gehäuse 74, 104, 116 bzw. 124 mit einem Schlauch nicht nur mit sich verändernder Wandstärke, sondern auch mit im wesentlichen konstanter Wandstärke eingesetzt werden kann. Als starres Gehäuse kann natürlich auch ein anderer im wesentlichen starrer Körper, beispielsweise ein Netz und dgl., eingesetzt werden.

**Patentansprüche**

1. Vorrichtung zum Reinigen von Blut von Stoffwechselprodukten, insbesondere für die Dialyse mit einem Anschluß (20) (Single-Needle-Dialyse) an den Blutkreislauf des Patienten, mit einer ersten von diesem Anschluß wegführenden Leitung (26) und einer zweiten zu diesem Anschluß führenden Leitung (30), mit einem Dialysator (40), der mit diesen Leitungen (26, 30) verbunden ist, mit wenigstens einer Pumpe (32, 36) und wenigstens einer Klemme (42, 84) an wenigstens einer der Leitungen (26, 30) zur Steuerung der Blutzufuhr und -abfuhr im extrakorporalen Kreislauf, einem in einer der Leitungen angeordneten erweiterbaren Speicher (34) für das Blut sowie mit einer Reglereinrichtung (90) zur Steuerung der Pumpe (32, 36) und des Absperrorgans (42, 84), dadurch gekennzeichnet, daß der Speicher (34) einen an seinem Außenumfang materialelastisch erweiterbaren und kontrahierbaren Schlauch (54, 76, 96, 106, 124) in einem Gehäuse (74, 104, 106) aufweist, das zwei, jeweils das Endes des Schlauchs (54, 76, 96, 106, 124) aufnehmende Anschlußstücke (46, 48, 66, 68, 110) und wenigstens eine Entlüftungsöffnung (80) aufweist.

2. Vorrichtung nach Anspruch 1, dadurch gekennzeichnet, daß der Schlauch (54, 76, 96, 106, 124) an seinen Enden erweitert und jeweils über eine Platte (50, 52, 62, 64, 98, 108) gespannt ist, die jeweils einen Anschluß (46, 48, 66, 68, 110) aufweist.

3. Vorrichtung nach Anspruch 2, dadurch gekennzeichnet, daß das Gehäuse (74) jeweils an seinen Enden an den Platten (62, 64, 98, 108) befestigt ist.

4. Vorrichtung nach Anspruch 1 oder 2, dadurch gekennzeichnet, daß der Schlauch (54, 76, 96, 106, 124) aus einem Material mit einer Bruchdehnung von wenigstens 200 % besteht und in seinem Volumen um wenigstens das Fünffache erweiterbar ist.

5. Vorrichtung nach Anspruch 4, dadurch gekennzeichnet, daß der Schlauch (54, 76, 96, 106, 124) aus Polyurethanen, Kautschuken, Silicon-Kautschuken, Latex, Gummi, regeneriertem Gummi oder Neoprenen besteht.

6. Vorrichtung nach Anspruch 1 oder 2, dadurch gekennzeichnet, daß die Wandstärke des Schlauchs (54, 76, 96, 106, 124) im wesentlichen über seine gesamte Länge gleich bleibt.

7. Vorrichtung nach Anspruch 1 oder 2, dadurch gekennzeichnet, daß die Wandstärke des Schlauchs (54, 76, 96, 106, 124) von seinem einen Ende zum anderen Ende stetig abnimmt.

8. Vorrichtung nach Anspruch 1 oder 2, dadurch gekennzeichnet, daß sich der Schlauch (54, 76, 96, 106, 124) oberhalb etwa 20 mm Hg ohne wesentlichen Druckanstieg erweitern läßt.

9. Vorrichtung nach Anspruch 1 oder 2, dadurch gekennzeichnet, daß der Schlauch (54, 76, 96, 106, 124) zwischen den Platten (60, 52, 62, 64, 98, 108) axial vorgespannt ist.

10. Vorrichtung nach Anspruch 1, dadurch gekennzeichnet, daß am Außenumfang des Gehäuses (74, 106, 116) optische und/oder mechanische Sensoren (86, 92) angeordnet sind.

11. Vorrichtung nach Anspruch 1, dadurch gekennzeichnet, daß stromauf des Speichers (34) die Pumpe (32) und stromab des Speichers (34) eine Klemme (84) angeordnet sind.

12. Vorrichtung nach Anspruch 1, dadurch gekennzeichnet, daß mit der Klemme (84) der im Dialysator (40) auftretende Druck im wesentlichen unabhängig vom Speicherdruck steuerbar ist.

## Claims

1. Device for purifying blood from products of metabolism, in particular for dialysis with a connection (20) (single-needle-dialysis) to the blood circulation of the patient, with a first line (26) leading away from this connection and a second line (30) leading to this connection, with a dialyser (40) which is connected to these lines (26, 30), with at least one pump (32, 36) and at least one clamp (42, 84) on at least one of the lines (26, 30) for controlling the blood supply and discharge in the extracorporeal circulation, an expandable store (34), located in one of the lines, for the blood and with a regulating device (90) for controlling the pump (32, 36) and the shut-off element (42, 84), characterised in that the store (34) has a hose (54, 76, 96, 106, 124), which is material-elastically expandable and contractable on its outer periphery, in a housing (74, 104, 106) which has two connectors (46, 48, 66, 68, 110), each receiving the end of the hose (54, 76, 96, 106, 124), and at least one vent orifice (80).

2. Device according to Claim 1, characterised in that the hose (54, 76, 96, 106, 124) is widened at its ends and is in each case fitted under tension over a plate (50, 52, 62, 64, 98, 108) which in each case has a connection (46, 48, 66, 68, 110).

3. Device according to Claim 2, characterised in that the housing (74) is fixed at each of its ends to the plates (62, 64, 98, 108).

4. Device according to Claim 1 or 2, characterised in that the hose (54, 76, 96, 106, 124) consists of a material having an elongation at break of at least 200 % and is expandable at least five-fold with respect to its volume.

5. Device according to Claim 4, characterised in that the hose (54, 76, 96, 106, 124) consists of polyurethanes, rubber types, silicon rubbers, latex, rubber, regenerated rubber or neoprenes.

6. Device according to Claim 1 or 2, characterised in that the wall thickness of the hose (54, 76, 96, 106, 124) remains substantially constant over its entire length.

7. Device according to Claim 1 or 2, characterised in that the wall thickness of the hose (54, 76, 96, 106, 124) decreases steadily from one of its ends to the other end.

8. Device according to Claim 1 or 2, characterised in that the hose (54, 76, 96, 106, 124) is expandable above about 20 mm Hg without a significant pressure rise.

9. Device according to Claim 1 or 2, characterised in that the hose (54, 76, 96, 106, 124) is axially pretensioned between the plates (60, 52, 62, 64, 98, 108).

10. Device according to Claim 1, characterised in that optical and/or mechanical sensors (86, 92) are provided on the outer periphery of the housing (74, 106, 116).

11. Device according to Claim 1, characterised in that the pump (32) is located upstream of the store (34) and a clamp (84) is located downstream of the store (34).

12. Device according to Claim 1, characterised in that the pressure arising in the dialyser (40) is controllable by the clamp (84), virtually independently of the store pressure.

## Revendications

1. Dispositif pour purifier le sang des produits du métabolisme basal, en particulier pour la dialyse, comportant un raccord (20) (dialyse à aiguille unique) dans le circuit sanguin du patient, comportant une première conduite (26) partant de ce raccord et une seconde conduite (30) conduisant à ce raccord, comportant un dialyseur (40) relié à ces conduites (26, 30), comportant au moins une pompe (32, 36) et au moins une pince (42, 84) sur au moins l'une des conduites (26, 30) pour commander l'admission et l'évacuation du sang dans le circuit extra-corporel, comportant un réservoir (34), extensible et disposé sur l'une des conduites, pour le sang, et comportant un dispositif de régulation (90) pour commander la pompe (32, 36) et l'organe de verrouillage (42, 84), caractérisé en ce que le réservoir (34) présente un tube souple (54, 76, 96, 106, 124) dont la périphérie extérieure peut se dilater et se contracter du fait de l'élasticité du matériau, dans un carter (74, 104, 106) qui présente deux raccords (46, 48, 66, 68, 110) recevant respectivement l'extrémité du tube souple (54, 76, 96, 106, 124) et au moins une ouverture de mise à l'atmosphère (80).

2. Dispositif selon la revendication 1, caractérisé en ce que l'on élargit le tube souple (54, 76, 96, 106, 124) à ses extrémités et qu'on le tend respectivement par dessus une plaque (50, 52, 62, 64, 98, 108) qui présente respectivement un raccord (46, 48, 66, 68, 110).

3. Dispositif selon la revendication 2, caractérisé en ce que le carter (74) est respectivement fixé à ses extrémités aux plaques (62, 64, 98, 108).

4. Dispositif selon la revendication 1 ou 2, caractérisé en ce que le tube souple (54, 76, 96, 016, 124) est constitué d'un matériau d'un allongement à la rupture d'au moins 200 % ; et en ce que son volume peut se dilater d'au moins cinq fois.

5. Dispositif selon la revendication 4, caractérisé en ce que le tube souple (54, 76, 96, 106, 124) est en polyuréthane, en caoutchouc synthétique, en caoutchouc de silicone, en latex, en caoutchouc naturel, en caoutchouc naturel régénéré ou en néoprène.

6. Dispositif selon la revendication 1 ou 2, caractérisé en ce que l'épaisseur de paroi du tube souple (54, 76, 96, 106, 124) reste sensiblement constante sur toute sa longueur.

7. Dispositif selon la revendication 1 ou 2, caractérisé en ce que l'épaisseur de paroi du tube souple (54, 76, 96, 106, 124) décroît de façon continue de l'une de ses extrémités à l'autre.

8. Dispositif selon la revendication 1 ou 2, caractérisé en ce que le tube souple (54, 76, 96, 106, 124) peut, au-dessus d'une pression d'environ 20 mm Hg, se dilater sans accroissement notable de pression.

9. Dispositif selon la revendication 1 ou 2, caractérisé en ce que le tube souple (54, 76, 96, 106, 124) est précontraint axialement entre les plaques (60, 52, 62, 64, 98, 108).

10. Dispositif selon la revendication 1, caractérisé en ce que des détecteurs optiques et/ou mécaniques (86, 92) sont disposés à la périphérie extérieure du carter (74, 106, 116).

11. Dispositif selon la revendication 1, caractérisé en ce que la pompe (32) est disposée en amont du réservoir (34) et une pince (84), en aval du réservoir (34).

12. Dispositif selon la revendication 1, caractérisé en ce qu'avec la pince (84) on peut commander la pression qui apparaît dans le dialyseur (40) essentiellement indépendamment de la pression qui règne dans le réservoir.

Fig. 1

## Fig. 2

# Fig. 3

Fig. 4

Fig. 5

Fig.6

Fig.7

Fig.8